# FASCICULE DE BREVET EUROPEEN

(11) **EP 0 920 299 B1**
(45) Date de publication et mention de la délivrance du brevet: **20.03.2002**
(21) Numéro de dépôt: 97938946.7
(22) Date de dépôt: 22.08.1997
(51) Int. Cl.: A61K 7/13

(54) **COMPOSITIONS DE TEINTURE DES FIBRES KERATINIQUES CONTENANT DES PYRROLO-OXAZOLES; LEUR UTILISATION POUR LA TEINTURE COMME COUPLEURS, PROCEDE DE TEINTURE**
PYRROLO-OXAZOL-ENTHALTENDE ZUSAMMENSETZUNGEN ZUR FÄRBUNG VON KERATINFASERN; IHRE VERWENDUNG ALS FARBSTOFFKOPPLER, FÄRBEVERFAHREN
COMPOSITIONS CONTAINING PYRROLO-OXAZOLES FOR DYEING KERATIN FIBRES; THEIR USE FOR DYEING AS COUPLERS, METHOD OF DYEING

(30) Priorité: 26.08.1996 FR 9610447
(43) Date de publication de la demande: 09.06.1999
(73) Titulaire: L'OREAL, 75008 Paris (FR)
(72) Inventeur: VIDAL, Laurent, F-75013 Paris (FR); MALLE, Gérard, F-77580 Villiers-sur-Morin (FR)
(74) Mandataire: Goulard, Sophie
(86) Numéro de dépôt international: FR9701522
(87) Numéro de publication internationale: WO9808487

(56) Documents cités:
- Aucun document pertinent relevé

## Description

L'invention a pour objet une composition pour la teinture d'oxydation des fibres kératiniques, en particulier des fibres kératiniques humaines telles que les cheveux, contenant à titre de coupleur au moins un composé pyrrolo-oxazole et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet, différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant, à titre de coupleurs, des composés pyrrolo-oxazoles en présence d'une base d'oxydation.

Cette découverte est à la base de la présente invention.

L'invention a pour objet une composition pour la teinture d'oxydation des fibres kératiniques et en particulier des fibres kératiniques humaines telles que les cheveux, caractérisée par le fait qu'elle comprend, dans un milieu approprié pour la teinture :
- à titre de coupleur, au moins un composé pyrrolo-oxazole de formule (I) suivante et/ou au moins l'un de leurs sels d'addition avec un acide : 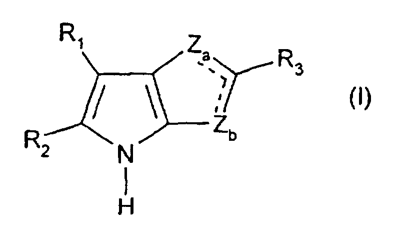 dans laquelle :
   - R₁ représente un atome d'hydrogène, un atome d'halogène tel que le brome, le chlore ou le fluor ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chlorobenzyloxy, méthoxyéthylcarbamoylméthoxy) ; un radical aryloxy (tel que par exemple : phénoxy, 4-méthoxyphénoxy, 4-nitrophénoxy, 4-cyanophénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyoxaloyloxy, pyruviloyloxy, cinnamoyloxy, myristoyloxy) ; un radical arylthio (tel que par exemple : phénylthio, 4-carboxy-phénylthio, 2-éthoxy 5-tert-butylphénylthio, 2-carboxyphénylthio, 4-méthane-sulfonyl-phénylthio); un radical alkylthio (tel que par exemple : méthylthio, éthylthio, propylthio, butylthio, 2-cyanoéthylthio, benzylthio, phénéthylthio, 2-(diéthylamino) éthylthio, éthoxyéthylthio, phénoxyéthylthio) ; un radical hétéroarylthio (tel que par exemple : 5-phényl 2,3,4,5-tétrazolylthio, 2-benzothiazolylthio); un radical hétéroaryloxy (tel que par exemple : 5-phényl 2,3,4,5-tétrazolyloxy, 2-benzo-thiazolyloxy) ; un radical thiocyano ; un radical alkyloxythiocarbonylthio (tel que dodécyloxythio carbonylthio) ; un radical sulfonamido (tel que benzènesulfonamido, N-éthyltoluène sulfonamido), un radical pentafluorobutanamido ; un radical 2,3,4,5,6-pentafluorobenzamido; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un radical alkylamido ; un radical arylamido ; un radical NR^{III}R^{IV} avec R^{III} et R^{IV} représentant, identiques ou différents, un alkyle en C₁-C₄, un hydroxyalkyle en C₁-C₄, un radical carboxyle, un radical alcoxycarboxyle, un radical alkyloxycarbonylamino, un radical aryloxycarbonylamino, un radical sulfonyloxy tel que méthanesulfonyloxy, un radical alcoxycarbonyloxy tel que methoxycarbonyloxy, éthoxycarbonyloxy
   - Zₐ et Z_{b}, différents l'un de l'autre, représentent un atome d'oxygène ou d'azote ;
   - R₂ et R₃, identiques ou différents, représentent un atome d'hydrogène ; un radical alkyle en C₁-C₂₀ linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyie, triazolyle, tétrazolyle, thiazolyle, oxazolyle, imidazolyle, ou thiadiazolyle), éventuellement substitué par 1 ou 2 radicaux R tels que définis précédemment ; un phényle substitué par un alkyle en C₁-C₄ ou un groupes trifluoromethyle,
      lorsque R₂ et/ou R₃ désigne un radical alkyle, un radical aryle ou un hétérocycle à 5 ou 6 chaînons (tels que définis ci-dessus), il peut être relié à l'atome de carbone du noyau par l'intermédiaire d'un atome d'oxygène, d'azote ou de soufre (dans ce cas, R₂ et/ou R₃ deviennent XR₂ et/ou XR₃ avec X = O, NH, S) ;
      R₂ et R₃ peuvent également désigner un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy ; un radical amino ; un radical acylamino ; un radical acyloxy ; un radical carbamoyloxy ; un radical sulfonamide ; un radical imide ; un radical uréido ; un radical sulfamoylamino ; un radical benzyle substitué par un alkyle en C₁-C₄ ou un trifluorométhyle ; un dialkylamino en C₁-C₄ ; un radical -(CH₂)ₚ-X-(CH₂)_{q}-OR' où p et q sont entiers, identiques ou différents, compris entre 1 et 3, R' represente H ou méthyle et X désigne un atome d'oxygène ou un groupe NR'' avec R'' designant hydrogène ou méthyle; un radical alcoxy carbonylamino ; un radical aryloxycarbonylamino ; un radical alcoxycarbonyle ; un radical aryloxycarbonyle ; un radical carboxyle; un groupe trifluorométhyle ;
   - et au moins une base d'oxydation

Les sels d'addition avec un acide des composés de l'invention peuvent être choisis notamment parmi les chlorhydrates, les bromhydrates, les tartrates, les tosylates, les benzènesulfonates, les sulfates, les lactates et les acétates.

Parmi les radicaux R₁ de la formule (I) définie ci-dessus, on préfère les radicaux choisis dans le groupe constitué par un atome d'hydrogène ; un radical alcoxy en C₁-C₄ phénoxy ; phénoxy substitué par un atome d'halogène, un alkyle en C₁-C₄, un carboxyle ou un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle ou un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ ou un hydroxyalkyle en C₁-C₄ ; un carboxyle ; un radical alcoxycarboxyle en C₁-C₄ ; un atome d'halogène tel que le chlore ou le brome.

Et encore plus particulièrement , on préfère les radicaux R₁ choisis dans le groupe constitué par hydrogène, chlore ; éthoxy ; phénoxy ; benzyloxy ; acyloxy ; acétamido et diméthylamino.

Parmi les radicaux R₂ et R₃ de la formule (I) définie ci-dessus, on préféré les radicaux choisis dans le groupe constitué par un atome d'hydrogène ; un alkyle en C₁-C₄, linéaire ou ramifié ; un phényle ; un phényle substitué par un atome d'halogène, un alkyle en C₁-C₄, un alcoxy en C₁-C₄, un groupe nitro, un groupe amino, un groupe trifluorométhyle ou alkylamino en C₁-C₄ ; un radical benzyle ; 3un radical benzyle substitué par un atome d'halogène, un alkyle en C₁-C₄, un alcoxy en C₁-C₄, un groupe nitro, un groupe amino, un groupe trifluorométhyle ; un alkylamino en C₁-C₄ ; un hétérocycle choisi parmi le thiophène, le furane ou la pyridine ; un radical trifluorométhyle ; un radical (CH₂)ₚ-X-(CH₂)_{q}-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C₄ ; un alkylamino en C₁-C₄ ; un dialkylamino en C₁-C₄ ; un arylamino ; un radical alcoxy choisi parmi méthoxy, éthoxy, phénoxy ; un halogène choisi parmi chlore, brome, fluor ; un groupe carboxyle ; un alcoxycarbonyle en C₁-C₄ ; un phényloxycarbonyle ; méthylthio ; éthylthio ; phénylthio ; méthanesulfonyle ; cyano.

Et encore plus particulièrement , on préfère les radicaux R₂ et R₃ de la formule (I) choisis dans le groupe constitué par hydrogène ; un alkyle choisi parmi méthyle, éthyle, isopropyle, ter-butyle ; un halogène choisi parmi fluor et chlore; phényle ; toluyle ; 4-chlorophényle ; 4-méthoxyphényle ; 3-méthoxyphényle ; 2-méthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

Et encore plus particulièrement, on préfère les radicaux R₂ et R₃ choisis dans le groupe constitué par un atome d'hydrogène ; un radical méthyle ; éthyle ; phényle ; toluyle ; 4-chlorophényle ; 4-méthoxyphényle ; benzyle ; trifluorométhyle ; chloro ; un radical méthoxy ou éthoxy ; un radical carboxyle ; méthylamino ou diméthylamino ; cyano.

Parmi les composés de formule (I) de l'invention préférentiels, on peut citer ceux choisis dans le groupe constitué par les composés de formule (I) dans lesquels R₁ représente un atome d'hydrogène ou de chlore et dans lesquels R₂ et R₃, identiques ou différents, représentent un atome d'hydrogène, un radical méthyle, éthyle, isopropyle, phényle, éthoxy, trifluorométhyle ou méthylthio.

Parmi les composés de formule (I) de l'invention plus particulièrement préférés, on peut citer ceux choisis dans le groupe constitué par :
- le 2-méthyl-6-phénylpyrrolo [3,2-d] oxazole ;
- le 2,6-diméthylpyrrolo [3,2-d] oxazole ;
- le 7-chloro-2-méthyl-6-phénylpyrrolo [3,2-d] oxazole ;
- le 8-méthyl-4-phénylpyrrolo [3,2-d] oxazole ;
- le 2-méthyl-6-phénylpyrrolo [3,2-d] oxazole ;
- le 4,8-diméthyl-6-phénylpyrrolo [3,2-d] oxazole ;

Les composés de formule (I) de la présente invention, leurs intermédiaires de synthèse et leurs procédés de préparation sont décrits dans la demande de brevet japonais JP 07 325 375, dans l'article J. Chem. Soc., 1948, 70, 2205 ; dans Synthesis, 1970, 648 ; dans les demandes de brevet FR 2 074 731 et EP 0879 953.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante, et leurs sels d'addition avec un acide : 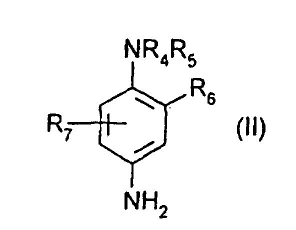 dans laquelle :
- R₄ représente un atome d'hydrogène, un radical alkyle en C₁-C₄, monohydroxyalkyle en C₁-C₄, polyhydroxyalkyle en C₂-C₄ ou alcoxy(C₁-C₄) alkyle(C₁-C₄),
- R₅ représente un atome d'hydrogène, un radical alkyle en C₁-C₄, monohydroxyalkyle en C₁-C₄ ou polyhydroxyalkyle en C₂-C₄,
- R₆ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
- R₇ représente un atome d'hydrogène ou un radical alkyle en C₁-C₄.

Dans la formule (II) ci-dessus, et lorsque R₆ est différent d'un atome d'hydrogène, alors R₄ et R₅ représentent de préférence un atome d'hydrogène et R₆ est de préférence identique à R₇, et lorsque R₆ représente un atome d'halogène, alors R₄, R₅ et R₇ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylène-diamine, la 2-β-hydroxyéthyl paraphénylènediamine, la 2-β-hydroxyéthyloxy para-phénylènediamine, la 2,6-diméthyl paraphénylènediamine, la 2,6-diéthyl paraphénylène-diamine, la 2,3-diméthyl paraphénylènediamine, la N,N-bis-(β-hydroxyéthyl) para-phénylènediamine, le 4-amino 1-(β-méthoxyèthyl)amino benzène, la 2-chloro para-phénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 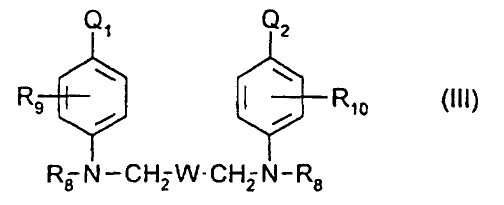 dans laquelle :
- Q₁ et Q₂, identiques ou différents, représentent un radical hydroxyle ou NHR₁₁ dans lequel R₁₁ représente un atome d'hydrogène ou un radical alkyle en C₁-C₄.
- R₈ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
- R₉ et R₁₀, identiques ou différents, représentent un atome d'hydrogène ou d'halogène ou un radical alkyle en C₁-C₄,
- W représente un radical pris dans le groupe constitué par les radicaux suivants : 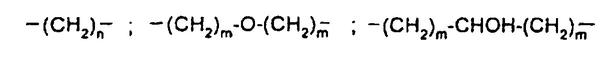 et 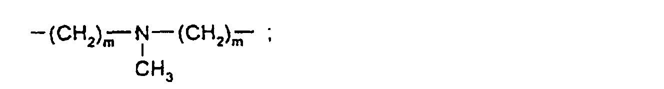
dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les paraaminophénols utilisables à titre de bases d'oxydation dans la composition tinctoriale selon l'invention, on peut notamment citer les composés répondant à la formule (IV) suivante, et leurs sels d'addition avec un acide : 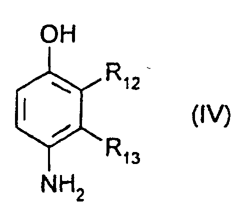 dans laquelle :
- R₁₂ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
- R₁₃ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₂ ou R₁₃ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triamino-pyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole et le 1-(4'-chlorobenzyl)-4,5-diaminopyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes -de sodium ou de potassium et les composés de formule (V) suivante : 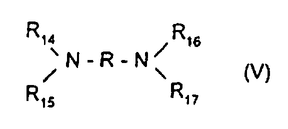 dans laquelle R est un reste propylène éventuellement substitué par un groupement hydroxyle ou un radical alkyle en C₁-C₄ ; R₁₄, R₁₅, R₁₆ et R₁₇, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des composés de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus.

Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES

### EXEMPLES 1 ET 2 DE PROCEDE TEINTURE EN MILIEU ALCALIN

On a préparé la composition tinctoriale, conformes à l'invention, suivante (teneurs en grammes) :
- 8-méthyl-4-phénylpyrrolo[3-2d] oxazole (coupleur) (^{*}) 0,594 g
- 4-(2-méthoxyéthylamino)aniline (base d'oxydation) 0,498 g
- Ethanol 20,0 g
- Ammoniaque à 20% de NH₃ 10,0 g
- Métabisulfite de sodium 0,228 g
- Agent séquestrant q.s
- Eau déminéralisée qsp 100 g
(^{*}) Le 8-méthyl-4-phénylpyrrolo [3-2d] oxazole est obtenu selon l'un des procédés de préparation décrits dans la demande de brevet japonais JP 07 325 375, dans l'article J. Chem. Soc., 1948, 70, 2205 ; dans Synthesis, 1970, 648 ; dans les demandes de brevet FR 2 074 731 et EP 0879 953.

### EXEMPLE 1

Au moment de l'emploi, la composition tinctoriale a été mélangée avec une quantité égale en poids d'une solution aqueuse de persulfate d'ammonium à 6.10-3 mole %.

Le mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage des mèches , on obtient des colorations puissantes, peu sélectives dans la gamme des rouges.

### EXEMPLE 2

Au moment de l'emploi, la composition tinctoriale a été mélangée avec une quantité égale en poids d'une solution aqueuse d'eau oxygénée à 20 volumes.

Le mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage des mèches , on obtient des colorations puissantes, peu sélectives dans la gamme des rouges.

### EXEMPLES 3 ET 4 DE PROCEDE TEINTURE EN MILIEU NEUTRE

On a préparé la composition tinctoriale, conforme à l'invention, suivante (teneurs en grammes) :
- 8-méthyl-4-phénylpyrrolo[3-2d] oxazole (coupleur) 0,594 g
- 4-(2-méthoxyéthylamino)aniline (base d'oxydation) 0,498 g
- Ethanol 20,0 g
- Tampon K₂HPO₄/KH₂PO₄ (1,5 M/1M) 10 g
- Métabisulfite de sodium 0,228 g
- Agent séquestrant q.s
- Eau déminéralisée qsp 100 g

### EXEMPLE 3

Au moment de l'emploi, la composition tinctoriale a été mélangée avec une quantité égale en poids d'une solution aqueuse de persulfate d'ammonium à 6.10⁻³ mole %.

Le mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage des mèches , on obtient des colorations puissantes, peu sélectives dans la gamme des rouges.

### EXEMPLE 4

Au moment de l'emploi, la composition tinctoriale a été mélangée avec une quantité égale en poids d'une solution aqueuse d'eau oxygénée à 20 volumes.

Le mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage des mèches , on obtient des colorations puissantes, peu sélectives dans la gamme des rouges.

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- à titre de coupleur, au moins un composé pyrrolo-oxazole de formule (I) suivante et/ou au moins l'un de leurs sels d'addition avec un acide : 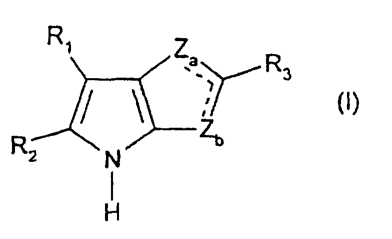
dans laquelle :
- R₁ représente : un atome d'hydrogène ; un atome d'halogène ; un radical alcoxy ; un radical aryloxy ; un radical acyloxy ; un radical arylthio ; un radical alkylthio ; un radical hétéroarylthio ; un radical hétéroaryloxy ; un radical thiocyano ; un radical alkyloxythiocarbonylthio ; un radical sulfonamido ; un radical pentafluorobutanamido ; un radical 2,3,4,5,6-pentafluorobenzamido ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un radical alkylamido; un radical arylamido ; un radical NR^{III}R^{IV} avec R^{III} et R^{IV} représentant, identiques ou différents, un alkyle en C₁-C₄, un hydroxyalkyle en C₁-C₄, un radical carboxyle, un radical alcoxycarboxyle, un radical alkyloxycarbonylamino, un radical aryloxycarbonylamino, un radical sulfonyloxy, un radical alcoxycarbonyloxy ou un radical aryloxycarbonyloxy ;
- Zₐ et Z_{b}, différents l'un de l'autre, représentent un atome d'oxygène ou d'azote ;
- R₂ et R₃, identiques ou différents, représentent un atome d'hydrogène ; un radical alkyle en C₁-C₂₀ linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle, éventuellement substitué par 1 ou 2 radicaux R tels que précédemment définis ; un hétérocycle à 5 ou 6 chaînons possédant au moins un atome d'azote, d'oxygène ou de soufre, éventuellement substitué par 1 ou 2 radicaux R tels que définis précédemment ; un phényle substitué par un alkyle en C₁-C₄ ou un trifluorométhyle ;
lorsque R₂ et/ou R₃ désigne un radical alkyle, un radical aryle ou un hétérocycle à 5 ou 6 chaînons (tels que définis ci-dessus), il peut être relié à l'atome de carbone du noyau par l'intermédiaire d'un atome d'oxygène, d'azote ou de soufre (dans ce cas, R₂ et/eu R₃ deviennent XR₂ et/ou XR₃ avec X = O, NH, S) ;
R₂ et R₃ peuvent également désigner un atome d'halogène ; un radical acyle ; un radical sulfonyle ; un radical sulfinyle ; un radical phosphonyle, un radical carbamoyle ; un radical sulfamoyle ; un radical cyano ; un radical siloxy ; un radical amino ; un radical acylamino ; un radical acyloxy ; un radical carbamoyloxy ; un radical sulfonamide ; un radical imide ; un radical uréido ; un radical sulfamoylamino ; un radical alcoxy carbonylamino ; un radical aryloxycarbonylamino ; un radical alcoxycarbonyle ; un radical aryloxycarbonyle ; un radical carboxyle ; un benzyle substitué par un alkyle en C₁-C₄ ou un trifluorométhyle ; un dialkylamino en C₁-C₄ ; un trifluorométhyle ; un radical -(CH₂)ₚ-X-(CH₂)_{q}-OR' où p et q, identiques ou différents, sont des entiers compris entre 1 et 3 ; R' représente H ou méthyle et X désigne un atome d'oxygène ou un groupe NR'' avec R'' désignant hydrogène ou méthyle ;
- et au moins une base d'oxydation.

2. Composition selon la revendication 1, **caractérisée par le fait que** les radicaux R, de la formule (I) sont choisis dans le groupe constitué par un atome d'hydrogène ; un radical alcoxy en C₁-C₄ ; phénoxy ; phénoxy substitué par un atome d'halogène, un alkyle en C₁-C₄, un carboxyle ou un groupe trifluorométhyle ; un radical acyloxy ; benzyloxy ; alkylthio en C₁-C₄; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ ou un hydroxyalkyle en C₁-C₄ ; un carboxyle ; un radical alcoxycarboxyle en C₁-C₄; un atome d'halogène.

3. Composition selon la revendication 1 ou 2, **caractérisée par le fait que** les radicaux R₁ de la formule (I) sont choisis dans le groupe constitué par hydrogène ; chlore ; éthoxy ; phénoxy ; benzyloxy ; acyloxy ; acétamido ; diméthylamino.

4. Composition selon l'une quelconque des revendications 1 à 3, **caractérisée par le fait que** les radicaux R₂ et R₃ de la formule (I) sont choisis dans le groupe constitué par un atome d'hydrogène ; un alkyle en C₁-C₄, linéaire ou ramifié ; un phényle ; un phényle substitué par un atome d'halogène, un alkyle en C₁-C_{4,} un alcoxy en C₁-C₄, un groupe nitro, un groupe amino, un groupe trifluorométhyle ou alkylamino en C₁-C₄ ; un radical benzyle ; un radical benzyle substitué par un atome d'halogène, un alkyle en C₁-C₄, un alcoxy en C₁-C₄, un groupe nitro, un groupe amino, un groupe trifluorométhyie ; un alkylamino en C₁-C₄ ; un hétérocycle choisi parmi le thiophène, le furane ou la pyridine ; un radical trifluorométhyfe ; un radical (CH₂)p-X-(CH₂)q-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C_{*4} ; un alkylamino en C₁-C₄ ; un dialkylamino en C₁-C₄ ; un arylamino ; un radical alcoxy choisi parmi méthoxy, éthoxy, phénoxy ; un halogène choisi parmi chlore, brome, fluor ; un groupe carboxyle ; un alcoxycarbonyle en C₁-C₄ ; un phényloxycarbonyle ; méthylthio ; éthylthio ; phénylthio ; méthanesulfonyle ; cyano.

5. Composition selon la revendication 4, **caractérisée par le fait que** les radicaux R₂ et R₃ de la formule (I) sont choisis dans le groupe constitué par un atome d'hydrogène ; un alkyle choisi parmi méthyle, éthyle, isopropyle, ter-butyle ; un halogène choisi parmi fluor et chlore ; phényle ; toluyle ; 4-chlorophényle ; 4-méthoxyphényle ; 3-méthoxyphényle ; 2-méthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

6. Composition selon la revendication 5, **caractérisée par le fait que** les radicaux R₂ et R₃ de la formule (I) sont choisis dans le groupe constitué par un atome d'hydrogène ; un radical méthyle ; éthyle ; phényle ; toluyle ; 4-chlorophényle ; 4-méthoxyphényle ; benzyle ; trifluorométhyle ; chloro ; un radical méthoxy ou éthoxy ; un radical carboxyle ; méthylamino ou diméthylamino ; cyano.

7. Composition selon l'une quelconque des revendications 1 à 6, **caractérisée par le fait que** les composés de formule (I) sont choisis dans le groupe constitué par les composés de formule (I) dans lesquels R₁ représente un atome d'hydrogène ou de chlore et dans lesquels R₂ et R₃, identiques ou différents, représentent un atome d'hydrogène, un radical méthyle, éthyle, isopropyle, phényle, éthoxy, trifluorométhyle ou méthylthio.

8. Composition selon l'une quelconque des revendications précédentes, **caractérisée par le fait que** les sels d'addition avec un acide des composés de formule (I) sont choisis parmi les chlorhydrates, les bromhydrates, les sulfates, les tartrates, les benzènesulfonates, les lactates, les tosylates et les acétates.

9. Composition selon l'une quelconque des revendications précédentes, **caractérisée par le fait que** le ou les composés de formule (I) sont choisis dans le groupe constitué par :
- le 2-méthyl-6-phénylpyrrolo [3,2-d] oxazole ;
- le 2,6-diméthylpyrrolo [3,2-d] oxazole ;
- le 7-chloro-2-méthyl-6-phénylpyrrolo [3,2-d] oxazole;
- le 8-méthyl-4-phénylpyrrolo [3,2-d] oxazole ;
- le 2-méthyl-6-phénylpyrrolo [3,2-d] oxazole ;
- le 4,8-diméthyl-6-phénylpyrrolo [3,2-d] oxazole ;

10. Composition selon l'une quelconque des revendications précédentes, **caractérisée par le fait que** le ou les composés de formule (I) représentent de 0,0005 à 12 % en poids du poids total de la composition tinctoriale.

11. Composition selon la revendication 10, **caractérisée par le fait que** le ou les composés de formule (I) représentent de 0,005 à 6 % en poids du poids total de la composition tinctoriale.

12. Composition selon l'une quelconque des revendications précédentes, **caractérisée par le fait que** la ou les bases d'oxydation sont choisies parmi les paraphénylènediamines, les bis-phénylalkylènediamines, les paraaminophénols, les ortho-aminophénols, les bases hétérocycliques, et leurs sels d'addition avec un acide.

13. Composition selon l'une quelconque des revendications précédentes, **caractérisée par le fait que** la ou les bases d'oxydation représentent de 0,0005 à 12% en poids environ du poids total de la composition tinctoriale.

14. Composition selon la revendication 13, **caractérisée par le fait que** la ou les bases d'oxydation représentent de 0,005 à 6 % en poids environ du poids total de la composition tinctoriale.

15. Composition selon l'une quelconque des revendications précédentes, **caractérisée par le fait qu'**elle renferme en outre un ou plusieurs coupleurs additionnels différents des composés de formule (I) et/ou un ou plusieurs colorants directs.

16. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcools inférieurs en C₁-C₄, le glycérol, les glycols et éthers de glycols, les alcools aromatiques, les produits analogues et leurs mélanges.

17. Composition selon l'une quelconque des revendications précédentes, **caractérisée par le fait qu'**elle présente un pH compris entre 3 et 12.

18. Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

19. Utilisation des composés de formule (I) ou de leurs sels d'addition avec un acide tels que définis dans l'une quelconque des revendications 1 à 9, à titre de coupleurs dans des compositions pour la teinture d'oxydation des fibres kératiniques et en particulier des fibres kératiniques humaines telles que les cheveux, en association avec au moins un base d'oxydation.

20. Procédé de teinture d'oxydation des fibres kératiniques et en particulier des fibres kératiniques humaines telles que les cheveux, **caractérisé par le fait qu'**on applique sur ces fibres au moins une composition tinctoriale telle que définie à l'une quelconque des revendication 1 à 18, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

21. Procédé selon la revendication 20, **caractérisé par le fait que** l'agent oxydant est choisi parmi le peroxyde d'hydrogène, le peroxyde d'urée, les bromates de métaux alcalins, les persels tels que les perborates et persulfates.

22. Dispositif à plusieurs compartiments, ou "kit" de teinture à plusieurs compartiments, dont un premier compartiment renferme une composition tinctoriale telle que définie à l'une quelconque des revendications 1 à 18 et un second compartiment renferme une composition oxydante.

## Patentansprüche

1. Zusammensetzung zum oxidativen Färben von Keratinfasern und insbesondere menschlichen Keratinfasern, wie dem Haar, **dadurch gekennzeichnet, daß** sie in einem zum Färben geeigneten Medium enthält:
- als Kuppler mindestens ein Pyrrolo-oxazol der folgenden Formel (I) und/oder mindestens ein Additionssalz dieser Verbindungen mit einer Säure: 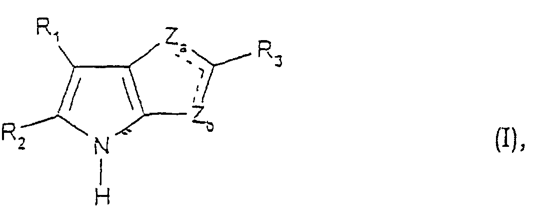 worin bedeuten :
- R₁ Wasserstoff; ein Halogenatom; Alkoxy; Aryloxy; Acyloxy; Arylthio; Alkylthio; Heteroarylthio; Heteroaryloxy; Thiocyano; Alkyloxythiocarbonylthio; Sulfonamido; Pentafluorbutanamido; 2,3,4,5,6-Pentafluorbenzamido; Pyrazolyl; Imidazolyl; Triazolyl; Tetrazolyl; Benzimidazolyl; 1-Benzyl-5-ethoxy-3-hydantoinyl; 1-Benzyl-3-hydantoinyl; 5,5-Dimethyl-2,4-dioxo-3-oxazolidinyl; 2-Oxy-1,2-dihydro-1-pyridinyl; Alkylamido; Arylamido; NR^{III}R^{IV}, worin R^{III} und R^{IV}, die identisch oder voneinander verschieden sind, C₁₋₄-Alkyl oder C₁₋₄-Hydroxyalkyl bedeuten, Carboxy, Alkoxycarboxy, Alkyloxycarbonylamino, Aryloxycarbonylamino, Sulfonyloxy, Alkoxycarbonyloxy oder Aryloxycarbonyloxy;
- Zₐ und Z_{b}, die identisch oder voneinander verschieden sind, ein Sauerstoffatom oder ein Stickstoffatom;
- die Gruppen R₂ und R₃, die identisch oder voneinander verschieden sind, Wasserstoff; eine geradkettige oder verzweigte C₁₋₂₀-Alkylgruppe, die gegebenenfalls mit einer oder zwei Gruppen R substituiert ist, die unter Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, Alkoxycarbonyl oder Acyl ausgewählt ist; eine Arylgruppe, die gegebenenfalls mit einer oder zwei oben definierten Gruppen R substituiert ist; einen 5- oder 6-gliedrigen Heterocyclus, der mindestens ein Stickstoff-, Sauerstoff- oder Schwefelatom enthält und der gegebenenfalls mit einer oder zwei oben definierten Gruppen R substituiert ist; Phenylgruppen, die mit einem C₁₋₄-Alkyl oder Trifluormethyl substituiert sind;
wenn die Gruppe R₂ und/oder R₃ eine Alkylgruppe, eine Arylgruppe oder einen 5- oder 6-gliedrigen Heterocyclus bedeutet (wie oben definiert), kann sie über ein Sauerstoff-, Stickstoff- oder Schwefelatom an das Kohlenstoffatom des Rings gebunden sein (in diesem Fall wird aus der Gruppe R₁ und/oder R₃ eine Gruppe XR₂ und/oder XR₃ mit X = O, NH oder S);
R₂ und/oder R₃ können auch bedeuten: Halogen; Acyl; Sulfonyl; Sulfinyl; Phosphonyl; Carbamoyl; Sulfamoyl; Cyano; Siloxy; Amino; Acylamino; Acyloxy; Carbamoyloxy; Sulfonamido; Imido; Ureido; Sulfamoyl-amino; Alkoxycarbonylamino; Aryloxycarbonylamino; Alkoxycarbonyl; Aryloxycarbonyl; Carboxy; Benzylgruppen, die mit einem C₁₋₄-Alkyl oder Trifluormethyl substituiert sind; C₁₋₄-Dialkylamino; Trifluormethyl ; Gruppen (CH₂)ₚ-X-(CH₂)_{q}-OR', worin p und q, die identisch oder voneinander verschieden sind, ganze Zahlen in Bereich von 1 bis 3 bedeuten, R' H oder Methyl bedeutet und X ein Sauerstoffatom oder eine Gruppe NR" mit R" = Wasserstoff oder Methyl ist;
und
- mindestens eine Oxidationsbase.

2. Zusammensetzung nach Anspruch 1, **dadurch gekennzeichnet, daß** die Gruppen R₁ der Formel (I) unter Wasserstoff; C₁₋₄-Alkoxy; Phenoxy; Phenoxygruppen, die mit einem Halogenatom, C₁₋₄-Alkyl, Carboxy oder Trifluormethyl substituiert sind; Acyloxy; Benzyloxy; C₁₋₄-Alkylthio; Phenylthio; Phenylthiogruppen, die mit einem Halogenatom, C₁₋₄-Alkyl, Carboxy oder Trifluormethyl substituiert sind; C₁₋₄-Alkylamido; Phenylamido; NR^{III}R^{IV}, worin R^{III} und R^{IV}, die identisch oder voneinander verschieden sind, C₁-₄-Alkyl oder C₁₋₄-Hydroxyalkyl bedeuten; Carboxy; C₁₋₄-Alkoxycarboxy oder einem Halogenatom ausgewählt sind.

3. Zusammensetzung nach Anspruch 1 oder 2, **dadurch gekennzeichnet, daß** die Gruppen R₁ der Formel (I) unter Wasserstoff; Chlor; Ethoxy; Phenoxy; Benzyloxy; Acyloxy; Acetamido und Dimethylamino ausgewählt sind.

4. Zusammensetzung nach einem der Ansprüche 1 bis 3, **dadurch gekennzeichnet, daß** die Gruppen R₂ und R₃ der Formel (I) unter Wasserstoff; geradkettigen oder verzweigten C₁-₄-Alkylgruppen; Phenyl; Phenylgruppen, die mit einem Halogenatom, C₁-₄-Alkyl, C₁₋₄-Alkoxy, Nitro, Amino, Trifluormethyl oder C₁-₄-Alkylamino substituiert sind; Benzyl; Benzylgruppen, die mit einem Halogenatom, C₁-₄-Alkyl, C₁-₄-Alkoxy, Nitro, Amino oder Trifluormethyl substituiert sind; C₁-₄-Alkylamino; Heterocyclen, wie Thiophen, Furan oder Pyridin; Trifluormethyl; Gruppen (CH₂)ₚ-X-(CH₂)_{q}-OR', worin p und q, die identisch oder voneinander verschieden sind, ganze Zahlen in Bereich von 1 bis 3 bedeuten, R' H oder Methyl bedeutet und X ein Sauerstoffatom oder eine Gruppe NR" mit R" = Wasserstoff oder Methyl ist; C₁₋₄-Hydroxyalkyl; C₁-₄-Aminoalkyl; C₁₋₄-Alkylamino; C₁-₄-Dialkylamino; Arylamino; Alkoxygruppen, die unter Methoxy, Ethoxy oder Phenoxy ausgewählt sind; Halogenatomen, die unter Chlor, Brom oder Fluor ausgewählt sind; Carboxy; C₁-₄-Alkoxycarbonyl; Phenyloxycarbonyl; Methylthio; Ethylthio; Phenylthio; Methansulfonyl; und Cyano ausgewählt sind.

5. Zusammensetzung nach Anspruch 4, **dadurch gekennzeichnet, daß** die Gruppen R₂ und R₃ der Formel (I) unter Wasserstoff; Alkylgruppen, die unter Methyl, Ethyl, Isopropyl und tert.-Butyl ausgewählt sind; Halogenatomen, die Fluor und Chlor ausgewählt sind; Phenyl; Tolyl; 4-Chlorphenyl; 4-Methoxyphenyl; 3-Methoxyphenyl; 2-Methoxyphenyl; Benzyl; Heterocyclen, die unter Pyridyl, Furyl oder Thienyl ausgewählt sind; Trifluormethyl; Hydroxymethyl; Aminomethyl; Methoxy oder Ethoxy; Methylamino oder Ethylamino oder Dimethylamino; Carboxy; Methoxycarbonyl oder Ethoxycarbonyl; und Cyano ausgewählt sind.

6. Zusammensetzung nach Anspruch 5, **dadurch gekennzeichnet, daß** die Gruppen R₂ und R₃ der Formel (I) unter Wasserstoff; Methyl; Ethyl; Phenyl; Tolyl; 4-Chlorphenyl; 4-Methoxyphenyl; Benzyl; Trifluormethyl; Chlor; Methoxy oder Ethoxy; Carboxy; Methylamino oder Dimethylamino; oder Cyano ausgewählt sind.

7. Zusammensetzung nach einem der Ansprüche 1 bis 6, **dadurch gekennzeichnet, daß** die Verbindungen der Formel (I) unter den Verbindungen der Formel (I) ausgewählt sind, worin R₁ Wasserstoff oder Chlor und die Gruppen R₂ und R₃, die identisch oder voneinander verschieden sind, Wasserstoff, Methyl, Ethyl, Isopropyl, Phenyl, Ethoxy, Trifluormethyl oder Methylthio bedeuten.

8. Zusammensetzung nach einem der vorhergehenden Ansprüche, **dadurch gekennzeichnet, daß** die Additionssalze der Verbindungen der Formel (I) mit einer Säure unter den Hydrochloriden, Hydrobromiden, Sulfaten, Tartraten, Benzolsulfonaten, Lactaten, Tosylaten und Acetaten ausgewählt sind.

9. Zusammensetzung nach einem der vorhergehenden Ansprüche, **dadurch gekennzeichnet, daß** die Verbindungen der Formel (I) ausgewählt sind unter:
- 2-Methyl-6-phenylpyrrolo[3,2-d]oxazol;
- 2,6-Dimethyl-pyrrolo[3,2-d]oxazol;
- 7-Chor-2-methyl-6-phenylpyrrolo[3,2-d]oxazol;
- 8-Methyl-4-phenylpyrrolo[3,2-d]oxazol;
- 2-Methyl-6-phenylpyrrolo[3,2-d]oxazol; und
- 4, 8-Dimethyl-6 -phenylpyrrob [3, 2-d] oxazol.

10. Zusammensetzung nach einem der vorhergehenden Ansprüche, **dadurch gekennzeichnet, daß** die Verbindung(en) der Formel (I) 0,0005 bis 12 Gew.-% des Gesamtgewichts der Farbmittelzusammensetzung ausmachen.

11. Zusammensetzung nach Anspruch 10, **dadurch gekennzeichnet, daß** die Verbindung(en) der Formel (I) 0,005 bis 6 Gew.-% des Gesamtgewichts der Farbmittelzusammensetzung ausmachen.

12. Zusammensetzung nach einem der vorhergehenden Ansprüche, **dadurch gekennzeichnet, daß** die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

13. Zusammensetzung nach einem der vorhergehenden Ansprüche, **dadurch gekennzeichnet, daß** die Oxidationsbase(n) etwa 0,0005 bis 12 Gew.-% des Gesamtgewichts der Farbmittelzusammensetzung ausmachen.

14. Zusammensetzung nach Anspruch 13, **dadurch gekennzeichnet, daß** die Oxidationsbase(n) etwa 0,005 bis 6 Gew.-% des Gesamtgewichts der Farbmittelzusammensetzung ausmachen.

15. Zusammensetzung nach einem der vorhergehenden Ansprüche, **dadurch gekennzeichnet, daß** sie ferner einen oder mehrere zusätzliche Kuppler, die von den Verbindungen der Formel (I) verschieden sind, und/oder einen oder mehrere Direktfarbstoffe enthält.

16. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter den niederen C₁₋₄-Alkoholen, Glycerin, Glykolen und Glykolethern, aromatischen Alkoholen, analogen Produkten und deren Gemischen ausgewählt ist.

17. Zusammensetzung nach einem der vorhergehenden Ansprüche, **dadurch gekennzeichnet, daß** sie einen pH-Wert im Bereich von 3 bis 12 aufweist.

18. Zusammensetzung nach einem der vorhergehenden Ansprüche, **dadurch gekennzeichnet, daß** sie als Flüssigkeit, Creme oder Gel oder in beliebigen weiteren Formen, die zur Durchführung einer Färbung von Keratinfasern und insbesondere von menschlichem Haar geeignet sind, vorliegt.

19. Verwendung von Verbindungen der Formel (I) oder von Additionssalzen dieser Verbindungen mit einer Säure nach einem der Ansprüche 1 bis 9 als Kuppler in Zusammensetzungen zum oxidativen Färben von Keratinfasern und insbesondere menschlichen Keratinfasern, wie dem Haar, in Kombination mit mindestens einer Oxidationsbase.

20. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 18 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

21. Verfahren nach Anspruch 20, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

22. Vorrichtung mit mehreren Abteilungen oder "Kit" zum Färben mit mehreren Abteilungen, wobei eine erste Abteilung eine Farbmittelzusammensetzung nach einem der Ansprüche 1 bis 18 und eine zweite Abteilung eine oxidierende Zusammensetzung enthält.

## Claims

1. Composition for the oxidation dyeing of keratinous fibres and in particular of human keratinous fibres, such as hair, **characterized in that** it comprises, in a medium appropriate for dyeing:
- as coupler, at least one pyrrolooxazole compound of following formula (I) and/or at least one of their addition salts with an acid: 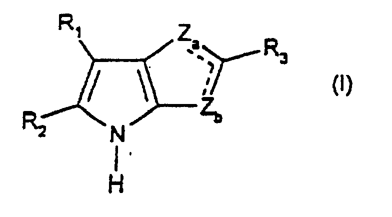
in which:
- R₁ represents: a hydrogen atom; a halogen atom; an alkoxy radical; an aryloxy radical; an acyloxy radical; an arylthio radical; an alkylthio radical; a heteroarylthio radical; a heteroaryloxy radical; a thiocyano radical; an alkyloxythiocarbonylthio radical; a sulphonamido radical; a pentafluorobutanamido radical; a 2,3,4,5,6-pentafluorobenzamido radical; a pyrazolyl radical; an imidazolyl radical; a triazolyl radical; a tetrazolyl radical; a benzimidazolyl radical; a 1-benzyl-5-ethoxy-3-hydantoinyl radical; a 1-benzyl-3-hydantoinyl radical; a 5,5-dimethyl-2,4-dioxo-3-oxazolidinyl radical; a 2-oxy-1,2-dihydro-1-pyridinyl radical; an alkylamido radical; an arylamido radical; an NR^{III}R^{IV} radical with R^{III} and R^{IV}, which are identical or different, representing a C₁-C₄ alkyl or a C₁-C₄ hydroxyalkyl; a carboxyl radical; an alkoxycarbonyl radical; an alkyloxycarbonylamino radical; an aryloxycarbonylamino radical; a sulphonyloxy radical; an alkoxycarbonyloxy radical; or an aryloxycarbonyloxy radical;
- Zₐ and Z_{b}, which are different from one another, represent an oxygen or nitrogen atom;
- R₂ and R₃, which are identical or different, represent a hydrogen atom; a linear or branched C₁-C₂₀ alkyl radical, optionally substituted by 1 or 2 R radicals chosen from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, alkoxycarbonyl or acyl; an aryl radical, optionally substituted by 1 or 2 R radicals as defined above; or a. 5- or 6-membered heterocycle having at least one nitrogen, oxygen or sulphur atom, optionally substituted by 1 or 2 R radicals as defined above; a phenyl substituted by a C₁-C₄ alkyl or a trifluoromethyl group ;
when R₂ and/or R₃ denotes an alkyl radical, an aryl radical or a 5- or 6-membered heterocycle (as defined above), it can be connected to the carbon atom of the nucleus via an oxygen, nitrogen or sulphur atom (in this case, R₂ and/or R₃ become XR₂ and/or XR₃, with X = O, NH or S);
R₂ and R₃ can also denote a halogen atom; an acyl radical; a sulphonyl radical; a sulphinyl radical; a phosphonyl radical; a carbamoyl radical; a sulphamoyl radical; a cyano radical; a siloxy radical; an amino radical; an acylamino radical; an acyloxy radical; a carbamoyloxy radical; a sulphonamido radical; an imido radical; a ureido radical; a sulphamoylamino radical; an alkoxycarbonylamino radical; an aryloxycarbonylamino radical; an alkoxycarbonyl radical; an aryloxycarbonyl radical; or a carboxyl radical; a benzyl radical substituted by a a C₁-C₄ alkyl or a trifluoromethyl group; a C₁-C₄ dialkylamino; a trifluoromethyl radical; a (CH₂)ₚ-X-(CH₂)_{q}-OR' radical, where p and q are identical or different integers between 1 and 3, R' represents H or methyl and X denotes an oxygen atom or an NR" group with R" denoting hydrogen or methyl;
- and at least one oxidation base.

2. Composition according to Claim 1, **characterized in that** the R₁ radicals of the formula (I) are chosen from the group consisting of a hydrogen atom; a C₁-C₄ alkoxy radical; a phenoxy radical; a phenoxy radical substituted by a halogen atom, a C₁-C₄ alkyl, a carboxyl or a trifluoromethyl group; an acyloxy radical; a benzyloxy radical; a C₁-C₄ alkylthio radical; a phenylthio radical; a phenylthio radical substituted by a halogen atom, a C₁-C₄ alkyl, a carboxyl or a trifluoromethyl group; a C₁-C₄ alkylamido radical; a phenylamido radical; an NR^{III} R^{IV} radical with R^{III} and R^{IV}, which are identical or different, representing a C₁-C₄ alkyl or a C₁-C₄ hydroxyalkyl; a carboxyl; a C₁-C₄ alkoxycarbonyl radical; or a halogen atom.

3. Composition according to Claim 1 or 2, **characterized in that** the R₁ radicals of the formula (I) are chosen from the group consisting of hydrogen, chlorine, ethoxy, phenoxy, benzyloxy, acyloxy, acetamido or dimethylamino.

4. Composition according to any one of Claims 1 to 3, **characterized in that** the R₂ and R₃ radicals of the formula (I) are chosen from the group consisting of a hydrogen atom; a linear or branched C₁-C₄ alkyl; a phenyl; a phenyl substituted by a halogen atom, a C₁-C₄ alkyl, a C₁-C₄ alkoxy, a nitro group, an amino group, a trifluoromethyl group or a C₁-C₄ alkylamino group; a benzyl radical; a benzyl radical substituted by a halogen atom, a C₁-C₄ alkyl, a C₁-C₄ alkoxy, a nitro group, an amino group or a trifluoromethyl group; a C₁-C₄ alkylamino; a heterocycle chosen from thiophene, furan or pyridine; a trifluoromethyl radical; a (CH₂)ₚ-X-(CH₂)_{q}-OR' radical, where p and q are identical or different integers between 1 and 3, R' represents H or methyl and X denotes an oxygen atom or an NR" group with R" denoting hydrogen or methyl; a C₁-C₄ hydroxyalkyl; a C₁-C₄ aminoalkyl; a C₁-C₄ alkylamino; a C₁-C₄ dialkylamino; an arylamino; an alkoxy radical chosen from methoxy, ethoxy or phenoxy; a halogen chosen from chlorine, bromine or fluorine; a carboxyl group; a C₁-C₄ alkoxycarbonyl; a phenyloxycarbonyl; methylthio; ethylthio; phenylthio; methanesulphonyl; or cyano.

5. Composition according to Claim 4, **characterized in that** the R₂ and R₃ radicals of the formula (I) are chosen from the group consisting of a hydrogen atom; an alkyl chosen from methyl, ethyl, isopropyl or tert-butyl; a halogen chosen from fluorine and chlorine; phenyl; toluyl; 4-chlorophenyl; 4-methoxyphenyl; 3-methoxyphenyl; 2-methoxyphenyl; benzyl; a heterocycle chosen from pyridyl, furyl or thienyl; trifluoromethyl; hydroxymethyl; aminomethyl; methoxy or ethoxy; methylamino or ethylamino or dimethylamino; carboxyl; methoxycarbonyl or ethoxycarbonyl; or cyano.

6. Composition according to Claim 5, **characterized in that** the R₂ and R₃ radicals of the formula (I) are chosen from the group consisting of a hydrogen atom; a methyl radical; ethyl; phenyl; toluyl; 4-chlorophenyl; 4-methoxyphenyl; benzyl; trifluoromethyl; chloro; a methoxy or ethoxy radical; a carboxyl radical; methylamino or dimethylamino; or cyano.

7. Composition according to any one of Claims 1 to 6, **characterized in that** the compounds of formula (I) are chosen from the group consisting of the compounds of formula (I) in which R₁ represents a hydrogen or chlorine atom and in which R₂ and R₃, which are identical or different, represent a hydrogen atom or a methyl, ethyl, isopropyl, phenyl, ethoxy, trifluoromethyl or methylthio radical.

8. Composition according to any one of the preceding claims, **characterized in that** the addition salts with an acid of the compounds of formula (I) are chosen from hydrochlorides, hydrobromides, sulphates, tartrates, benzenesulphonates, lactates, tosylates and acetates.

9. Composition according to any one of the preceding claims, **characterized in that** the compound or compounds of formula (I) are chosen from the group consisting of:
- 2-methyl-6-phenylpyrrolo[3,2-d]oxazole;
- 2,6-dimethylpyrrolo[3,2-d]oxazole;
- 7-chloro-2-methyl-6-phenylpyrrolo[3,2-d]oxazole;
- 8-methyl-4-phenylpyrrolo[3,2-d]oxazole;
- 2-methyl-6-phenylpyrrolo[3,2-d]oxazole;
- 4,8-dimethyl-6-phenylpyrrolo[3,2-d]oxazole;

10. Composition according to any one of the preceding claims, **characterized in that** the compound or compounds of formula (I) represent from 0.0005 to 12% by weight of the total weight of the dyeing composition.

11. Composition according to Claim 10, **characterized in that** the compound or compounds of formula (I) represent from 0.005 to 6% by weight of the total weight of the dyeing composition.

12. Composition according to any one of the preceding claims, **characterized in that** the oxidation base or bases are chosen from para-phenylenediamines, bisphenylalkylenediamines, para-aminophenols, ortho-aminophenols, heterocyclic bases, and their addition salts with an acid.

13. Composition according to any one of the preceding claims, **characterized in that** the oxidation base or bases represent from 0.0005 to 12% by weight approximately of the total weight of the dyeing composition.

14. Composition according to Claim 13, **characterized in that** the oxidation base or bases represent from 0.005 to 6% by weight approximately of the total weight of the dyeing composition.

15. Composition according to any one of the preceding claims, **characterized in that** it furthermore includes one or more additional couplers other than the compounds of formula (I) and/or one or more direct dyes.

16. Composition according to any one of the preceding claims, **characterized in that** the medium (or vehicle) appropriate for dyeing is composed of water or of a mixture of water and of at least one organic solvent chosen from lower C₁-C₄ alcohols, glycerol, glycols and glycol ethers, aromatic alcohols, analogous products and their mixtures.

17. Composition according to any one of the preceding claims, **characterized in that** it exhibits a pH of between 3 and 12.

18. Composition according to any one of the preceding claims, **characterized in that** it is provided in the form of liquids, creams or gels or in any other form appropriate for carrying out dyeing of keratinous fibres, and in particular of human hair.

19. Use of the compounds of formula (I) or of their addition salts with an acid as defined in any one of Claims 1 to 9, as couplers, in combination with at least one oxidation base, in compositions for the oxidation dyeing of keratinous fibres and in particular of human keratinous fibres, such as hair.

20. Process for the oxidation dyeing of keratinous fibres and in particular of human keratinous fibres, such as hair, **characterized in that** at least one dyeing composition as defined in any one of Claims 1 to 18 is applied to these fibres, the colour being developed at acidic, neutral or alkaline pH using an oxidizing agent which is added only at the time of use to the dyeing composition or which is present in an oxidizing composition applied simultaneously or sequentially in a separate fashion.

21. Process according to Claim 20, **characterized in that** the oxidizing agent is chosen from hydrogen peroxide, urea hydrogen peroxide, alkali metal bromates, or persalts, such as perborates and persulphates.

22. Dyeing multi-compartment device or kit with several compartments, a first compartment of which includes a dyeing composition as defined in any one of Claims 1 to 18 and a second compartment of which includes an oxidizing composition.
